# EUROPEAN PATENT SPECIFICATION

(11) **EP 4 275 666 B1**
(45) Date of publication and mention of the grant of the patent: **03.12.2025**
(21) Application number: 22780008.3
(22) Date of filing: 11.03.2022
(51) Int. Cl.: A61F 13/496, A61F 13/49, A61F 13/532, A61F 13/535

(54) **PANTS-TYPE ABSORBENT ARTICLE**
HOSENARTIGER SAUGFÄHIGER ARTIKEL
ARTICLE ABSORBANT DE TYPE CULOTTE

(30) Priority: 31.03.2021 JP 2021061140
(43) Date of publication of application: 15.11.2023
(73) Proprietor: Unicharm Corporation, Ehime 799-0111 (JP)
(72) Inventor: MUKAI, Hirotomo, Kanonji-shi, Kagawa 769-1602 (JP)
(74) Representative: Dolleymores
(86) International application number: PCT/JP2022/011061
(87) International publication number: WO 2022/209787

(56) References cited:
- EP-A1- 1 153 585
- EP-A1- 3 646 833
- WO-A1-2007/074682
- WO-A1-2020/241024
- JP-A- 2019 030 551
- JP-A- 2019 118 557
- JP-A- 2020 192 123
- US-B2- 10 813 796

## Description

### FIELD

The present invention relates to an underpants-shaped absorbent article.

### BACKGROUND

Patent Literature 1 discloses an underpants-shaped absorbent article that includes an absorbent main body, a front waist member, and a back waist member, and in which the front waist member and the back waist member are joined by a pair of joining portions on two lateral side portions. The back waist member has an extension portion that extends downward beyond the joining portion, and the extension portion can cover the wearer's buttocks. Further, the extension portion includes inclined elastic members arranged in lateral side end portions with being inclined with respect to the lateral direction, and lateral elastic members arranged along the lateral direction. Therefore, the extension portion is likely to maintain in a state of fitting along the outline of the wearer's buttocks without turn-up or positional deviation. EP3646833A1 discloses an underpants-shaped absorbent article that includes an absorbent main body, a front waist member and a back waist member.

### [CITATION LIST]

### [PATENT LITERATURE]

Patent Literature 1: Japanese Patent Application Publication No. 2019-30551

### SUMMARY

### [TECHNICAL PROBLEM]

Although the fit of the extension portion is enhanced by providing elastic members in the extension portion as described above, the absorbent core is irregularly deformed (contracted) due to the elastic members of the extension portion, causing a risk that a gap is formed between the wearer and the absorbent core to cause leakage of excrement. In particular, since the extension portion is positioned on the crotch side with respect to the waist portion, the proportion of a region that overlaps the absorbent core increases. That is, the elastic members provided in the extension portion are likely to affect the contraction of the absorbent core.

The present invention was achieved in light of problems that described above and an aspect of the present invention is to enhance the fit of an extension portion and an absorbent core to a wearer, while suppressing irregular deformation of the absorbent core due to elastic members provided in the extension portion.

### [SOLUTION TO PROBLEM]

A main aspect of the present invention of achieving the above-described aspect is an underpants-shaped absorbent article as defined in claim 1.

Features of the present invention other than the above will become clear by reading the present specification with reference to the accompanying drawings.

### [ADVANTAGEOUS EFFECT OF THE INVENTION]

According to the present invention, it is possible to enhance the fit of an extension portion and an absorbent core to a wearer, while suppressing irregular deformation of the absorbent core due to elastic members provided in the extension portion.

### BRIEF DESCRIPTION OF DRAWINGS

FIG. 1 is a front view of an underpants-shaped disposable diaper 1.
FIG. 2 is a schematic plan view of the diaper 1 in an unfolded and stretched state when viewed from a skin side.
FIG.3 is a schematic cross-sectional view taken along a line A-A in FIG.2.
FIG. 4 is an explanatory diagram illustrating a low-basis-weight region 40 of an absorbent core 12.
FIG. 5 is an explanatory diagram illustrating the low-basis-weight region 40 of the absorbent core 12.
FIGS. 6A and 6B are explanatory diagrams illustrating the absorbent core 12.
FIG. 7 is an explanatory diagram illustrating a diaper 1 of a modified example.
FIG. 8 is an explanatory diagram illustrating a diaper 1 of a second embodiment.
FIG. 9 is an explanatory diagram illustrating a diaper 1 of a third embodiment.
FIG. 10A is a schematic front view of the diaper 1 of the third embodiment in a natural state, and FIG. 10B is a schematic front view of the diaper 1 of the third embodiment in a put-on state.

### DESCRIPTION OF EMBODIMENTS

At least following matters will become clear with description of this specification and attached drawings.

An underpants-shaped absorbent article having a vertical direction, a lateral direction, and a front-back direction, the absorbent article including: an absorbent main body that includes an absorbent core; and an exterior member that is arranged on a non-skin side with respect to the absorbent main body, two lateral side portions of each of a pair of waist portions in the exterior member being joined by a pair of joining portions, the exterior member having an extension portion, the extension portion being a portion that extends downward from at least either one of the pair of waist portions, the extension portion having a string-like lateral elastic member that stretches and contracts in the lateral direction, and an inclined elastic member that stretches and contracts in a direction inclined with respect to the lateral direction, a plurality of low-basis-weight regions being provided in a portion of the absorbent core that overlaps the extension portion when the underpants-shaped absorbent article is in an unfolded state, the low-basis-weight region being a region in which a basis weight of the absorbent core is lower than in a surrounding region and that extends along at least either one direction of the vertical direction and the lateral direction.

According to the above-described underpants-shaped absorbent article, it is possible to contract the low-basis-weight regions due to the lateral elastic member and the inclined elastic member, making it possible to suppress irregular deformation of the absorbent core (e.g., deformation that the absorbent core protrudes toward the non-skin side). Further, when putting on the absorbent article, while expanding the contracted absorbent core due to the lateral elastic member and the inclined elastic member, the absorbent core (low-basis-weight region) can brought into close contact with the wearer's body. Therefore, the fit of the extension portion and the absorbent core to the wearer can be enhanced.

In such an underpants-shaped absorbent article, the lateral elastic member and the inclined elastic member are separate members and have an overlapping portion where the lateral elastic member and the inclined elastic member partially overlap each other in the front-back direction.

According to the above-described underpants-shaped absorbent article, the lateral elastic member is likely to stretch in conjunction with the stretching of the inclined elastic member. Therefore, the absorbent core (low-basis-weight regions) can be securely expanded in the lateral direction and brought into close contact with the wearer's body.

In such an underpants-shaped absorbent article, a total value of a force for stretching the inclined elastic member of the extension portion by a predetermined length in the inclined direction is greater than a total value of a force for stretching the lateral elastic member of the extension portion by the predetermined length in the lateral direction.

According to the above-described underpants-shaped absorbent article, the lateral elastic member is likely to stretch in conjunction with the stretching of the inclined elastic member having a high stretching stress. Therefore, the absorbent core (low-basis-weight regions) can be securely expanded in the lateral direction and brought into close contact with the wearer's body.

In such an underpants-shaped absorbent article, the exterior member has the extension portion that extends downward from a back waist portion.

According to the above-described underpants-shaped absorbent article, the rounded buttocks of the wearer can be covered with the extension portion, and the extension portion and the absorbent core can be brought into close contact along the buttocks that largely move.

In such an underpants-shaped absorbent article, a one-side portion of the exterior member that is located on an one side in the front-back direction has the extension portion, an other-side portion of the exterior member that is located on another side in the front-back direction has a rectangular shape in a stretched state, the absorbent main body has an exposed portion below the one-side portion of the exterior member in the front-back direction and below the other-side portion of the exterior member in the front-back direction, the exposed portion having a non-skin-side surface that is exposed to an outside, and a plurality of low-basis-weight regions are provided in a portion of the absorbent core that is located in the exposed portion when the underpants-shaped absorbent article is in the unfolded state, the low-basis-weight region being a region in which the basis weight of the absorbent core is lower than in a surrounding region and which extends along at least either one direction of the vertical direction and the lateral direction.

According to the above-described underpants-shaped absorbent article, the portion of the absorbent core that is positioned in the exposed portion is likely to bend along the wearer's crotch due to the low-basis-weight regions without being hindered by the exterior member. This makes it possible to suppress the leakage of excreted fluid.

In such an underpants-shaped absorbent article, the extension portion has a plurality of the lateral elastic members arranged side-by-side in the vertical direction, the low-basis-weight region has a longitudinally-elongated region that is longer in the vertical direction than in the lateral direction, and in the unfolded state of the underpants-shaped absorbent article, a vertical position of the longitudinally-elongated region overlaps vertical positions of two or more of the lateral elastic members.

According to the above-described underpants-shaped absorbent article, the portion of the absorbent core that overlaps the extension portion is likely to regularly contract across a wide range in the vertical direction, and likely to comes into close contact with the wearer's body in a state of expanding in the lateral direction.

In such an underpants-shaped absorbent article, the extension portion has a plurality of the lateral elastic members arranged side-by-side in the vertical direction, and a lower end of the inclined elastic member is located below the lowest lateral elastic member.

According to the above-described underpants-shaped absorbent article, it makes all of the plurality of lateral elastic members likely to stretch in conjunction with the stretching of the inclined elastic member, making the absorbent core (low-basis-weight region) possible to securely expand in the lateral direction and to come into close contact with the wearer's body.

In such an underpants-shaped absorbent article, the low-basis-weight region has a longitudinally-elongated region that is longer in the vertical direction than in the lateral direction and a laterally-elongated region that is longer in the lateral direction than in the vertical direction, and the longitudinally-elongated region and the laterally-elongated region are arranged intersecting each other.

According to the above-described underpants-shaped absorbent article, the absorbent core is likely to bend in various directions from the intersection portion of the longitudinally-elongated region and the laterally-elongated region, making the absorbent core likely to bend along the wearer's body.

In such an underpants-shaped absorbent article, a basis weight of the absorbent body including the absorbent core is 400 g/m² or more.

According to the above-described underpants-shaped absorbent article, the absorption capacity is high, and the low-basis-weight regions can prevent the absorbent core from becoming stiff. This makes it possible to reduce discomfort while the absorbent article is put on.

In such an underpants-shaped absorbent article, the low-basis-weight region has a plurality of longitudinally-elongated regions that are longer in the vertical direction than in the lateral direction, the plurality of longitudinally-elongated regions are arranged side-by-side in the lateral direction with a space, the absorbent core has a plurality of compressed portions in which a part of the absorbent core is compressed in a thickness direction, and in a stretched state of the underpants-shaped absorbent article, an average length of the compressed portions in the lateral direction is smaller than an average space between the longitudinally-elongated regions arranged side-by-side in the lateral direction.

According to the above-described underpants-shaped absorbent article, it decreases the probability that the compressed portions are located at the boundary portion between the low-basis-weight region (longitudinally-elongated region) and a high-basis-weight region adjacent thereto. Therefore, the low-basis-weight region and the high-basis-weight region are less likely to be integrated. This allows the low-basis-weight region to contract by the elastic member, making it possible to suppress irregular deformation of the absorbent core.

In such an underpants-shaped absorbent article, the exterior member has a waist elastic member that stretches and contracts in the lateral direction, in the waist portion, and a plurality of low-basis-weight regions is provided in a portion of the absorbent core that overlaps the waist portion when the underpants-shaped absorbent article is in the unfolded state, the low-basis-weight region being a region in which the basis weight of the absorbent core is lower than in a surrounding region and that extends along at least either one direction of the vertical direction and the lateral direction.

According to the above-described underpants-shaped absorbent article, it is possible to suppress irregular deformation of the portion of the absorbent core that is positioned in the waist portion. Further, when putting on the absorbent article, while expanding the contracted absorbent core (low-basis-weight regions) in the lateral direction due to the waist elastic member, it allows the absorbent core to come into close contact with the wearer's body.

In such an underpants-shaped absorbent article, when the underpants-shaped absorbent article is in the unfolded state and in a stretched state, a total area of the low-basis-weight regions located in the portion of the absorbent core that overlaps the waist portion is larger than a total area of the low-basis-weight regions located in the portion of the absorbent core that overlaps the extension portion.

According to the above-described underpants-shaped absorbent article, the absorbent core is made likely to bend along the buttocks and the lower abdomen, which are more rounded than the waist, and the fit of the absorbent core is enhanced.

In such an underpants-shaped absorbent article, the low-basis-weight region has a laterally-elongated region that is longer in the lateral direction than in the vertical direction, and the laterally-elongated region is located in a portion of the absorbent core that overlaps a back waist portion when the underpants-shaped absorbent article is in the unfolded state.

According to the above-described underpants-shaped absorbent article, the absorbent core is likely to bend in the vertical direction along the vertical outline of the wearer's body (from the waist portion to the buttocks), and the fit of the absorbent core is enhanced.

In such an underpants-shaped absorbent article, the low-basis-weight region has a laterally-elongated region that is longer in the lateral direction than in the vertical direction, and a plurality of the laterally-elongated regions are located side-by-side in the vertical direction in a portion of the absorbent core that overlaps a front waist portion when the underpants-shaped absorbent article is in the unfolded state.

According to the above-described underpants-shaped absorbent article, the absorbent core is likely to bend in the vertical direction along the vertical outline of the wearer's body (from the waist portion to the lower abdomen), and the fit of the absorbent core is enhanced.

In such an underpants-shaped absorbent article, the absorbent main body has an exposed portion below a front portion of the exterior member and below a back portion of the exterior member, the exposed portion having a non-skin-side surface that is exposed to an outside, and the low-basis-weight region is provided vertically straddling a lower end of at least either one of the front portion of the exterior member and the back portion of the exterior member.

According to the above-described underpants-shaped absorbent article, at the boundary portion between the portion of the absorbent core that is covered with the exterior member and the portion of the absorbent core that is not covered with the exterior member and is likely to bend, it is possible to suppress abrupt changes in bending of the absorbent core, making the bending possible to be moderate.

### Embodiments

The following describes the underpants-shaped absorbent article according to the present embodiment by way of example of an underpants-shaped disposable diaper for adults. However, the underpants-shaped absorbent article according to the present embodiment is not limited to the above, and is also applicable to an underpants-shaped disposable diaper for infants, an underpants-shaped sanitary napkin, or the like.

### First Embodiment

### Basic constitution of underpants-shaped disposable diaper 1

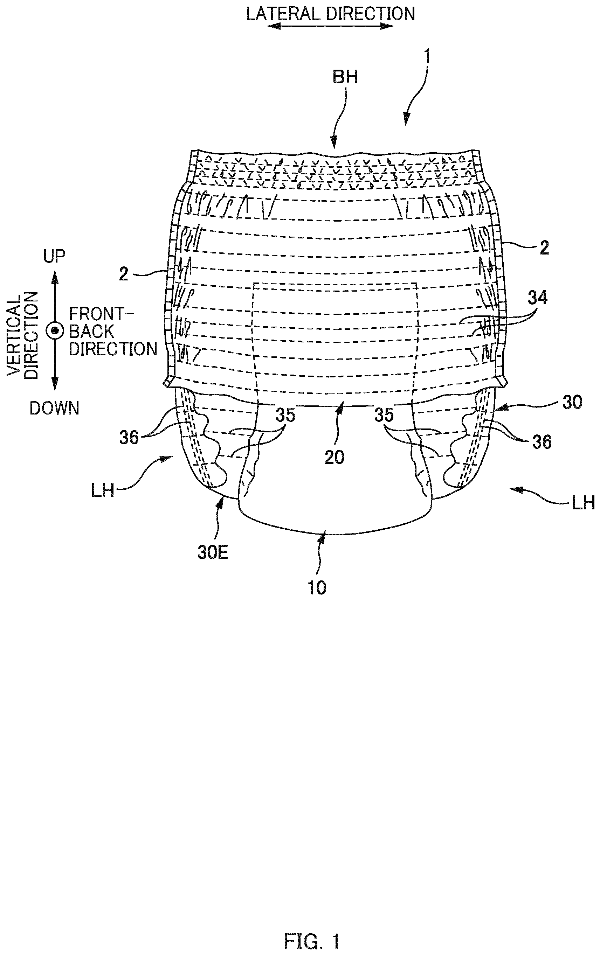

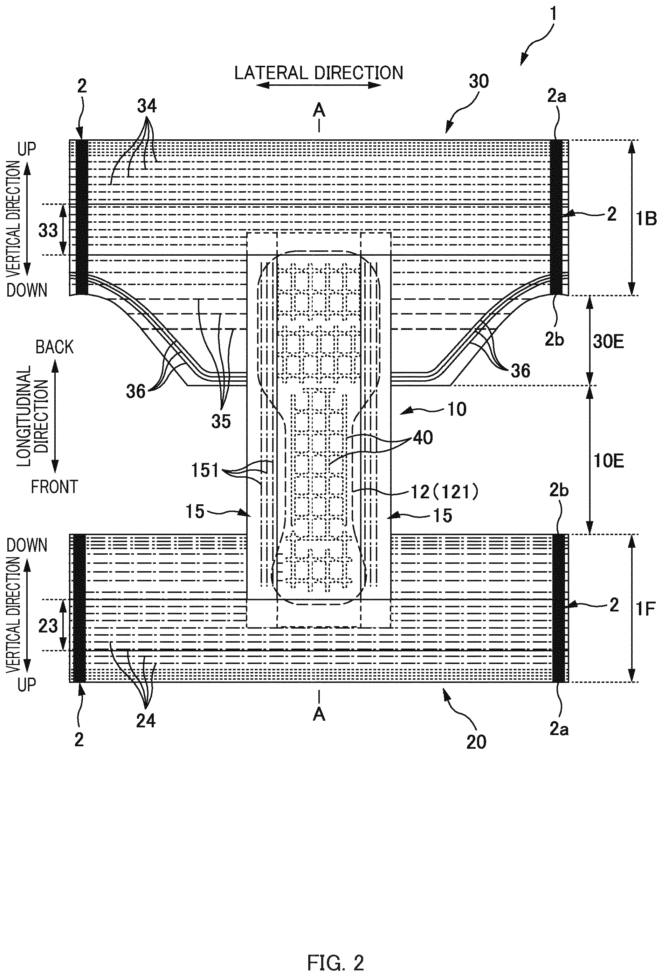

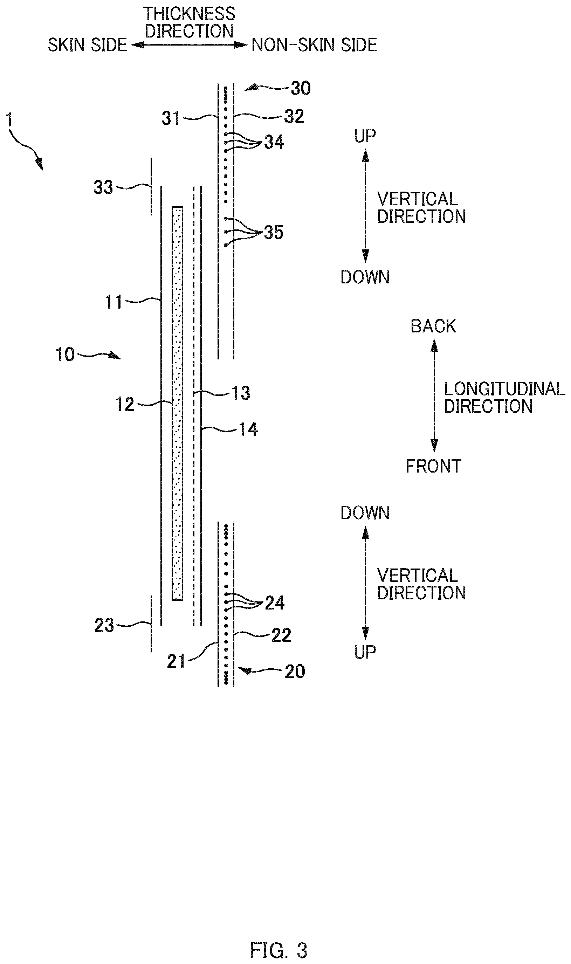

FIG. 1 is a front view of an underpants-shaped disposable diaper 1 (hereinafter, also referred to as a "diaper 1"). FIG. 2 is a schematic plan view of the diaper 1 in an unfolded and stretched state when viewed from a skin side. FIG.3 is a schematic cross-sectional view taken along a line A-A in FIG.2.

The unfolded state of the diaper 1 refers to a state where a pair of joining portions 2 provided on two side portions of the underpants-shaped diaper 1 are separated apart and the diaper 1 is spread out and unfolded flat. The stretched state of the diaper 1 refers to a state where the elastic members included in the diaper 1 are stretched to an extent that wrinkles in the diaper 1 are no longer visible. Specifically, the diaper 1 is stretched until the dimensions of the members that constitutes the diaper 1 match or are close to the dimensions of the members on their own (that is, the dimensions in a state where the stretchability of the elastic members is not exhibited).

As shown in FIG.1, the underpants-shaped diaper 1 has a vertical direction, a lateral direction, and a front-back direction, and the diaper 1 has a waist opening BH and a pair of leg openings LH. In the front-back direction, the side corresponding to the wearer's stomach is the front side, and the side corresponding to the wearer's back is the back side. Further, the diaper 1 has a thickness direction as shown in FIG. 3. With respect to the thickness direction, the side that comes into contact with the wearer is the skin side, and the side opposite to the skin side is the non-skin side.

The diaper 1 has an absorbent body 10 and exterior members 20 and 30 disposed on the non-skin side with respect to the absorbent body 10. The diaper 1 of the present embodiment is of a so-called three-piece type, and the exterior member has a front exterior member 20 and a back exterior member 30. The absorbent main body 10 has an exposed portion 10E whose non-skin-side surface is exposed to the outside, below the front exterior member 20 (the front portion of the exterior member) and below the back exterior member 30 (the back portion of the exterior member).

As shown in FIG.2, the non-skin-side surface of the longitudinal front end portion of the absorbent body 10 is joined to the skin-side surface of the front exterior member 20 with an adhesive or the like, and the non-skin-side surface of the longitudinal back end portion of the absorbent body 10 is joined to the skin-side surface of the back exterior member 30 with an adhesive or the like. From the diaper 1 in the unfolded state of FIG.2, the absorbent main body 10 is folded one time such that the front exterior member 20 and the back exterior member 30 face each other, and two lateral side portions of the front exterior member 20 and two lateral side portions of the back exterior member 30 are joined by welding (e.g., heat welding or ultrasonic welding) or the like, forming a pair of joining portions 2. Thereby, the diaper 1 in an underpants-shaped state is obtained.

In the present embodiment, in the diaper 1, a region located in the vertical range where the joining portions 2 extend (that is, a region from the position of an upper end 2a to the position of a lower end 2b of the joining portion 2) is referred to as "a pair of waist portions 1F and 1B". The waist portion located on the front side in the front-back direction will be referred to as a "front waist portion 1F" and the waist portion located on the back side will be referred to as a "back waist portion 1B".

The absorbent main body 10 includes a liquid-permeable top sheet 11, an absorbent body 12 that absorbs excrement, a liquid-impermeable back sheet 13, and an exterior sheet 14. They adhere with an adhesive or the like. The absorbent body 12 includes an absorbent core 121, and a liquid-permeable core-wrapping sheet 122 (tissue paper, nonwoven fabric, or the like) that covers the absorbent core 121. One example of the absorbent core 121 is an absorbent core obtained by shaping a liquid absorbent fibers (e.g., a pulp fiber) containing a superabsorbent polymer into a predetermined shape. Alternatively, the absorbent core 121 may be an SAP sheet in which an SAP layer is adhered to a hydrophilic sheet, an air-laid sheet in which liquid absorbent fibers are formed into a sheet by an air-laid method, and the like. Note that the absorbent body 12 does not have to include the core-wrapping sheet 122.

In addition, the absorbent body 10 has leak-proof wall portions 15 that are capable of rising up toward the skin side, in two lateral side portions. For example, the leak-proof wall portions 15 are formed as follow: two lateral side portions of the exterior sheet 14 are folded back laterally inward so as to be positioned on the skin-side surface of the top sheet 11, and the leak-proof-wall elastic members 151 (e.g., elastic strings) that stretch and contract in the longitudinal direction are provided on the folded-back portions.

As shown in FIG. 3, the front exterior member 20 and the back exterior member 30 respectively include: skin-side sheets 21 and 31 and non-skin-side sheets 22 and 32 which are formed of soft nonwoven fabric or the like; cover sheets 23 and 33 that cover the longitudinal ends of the absorbent main body 10 from the skin side; and a plurality of waist elastic members 24 and 34 that stretch and contract in the lateral direction (e.g., string-like elastic members such as elastic strings). In the waist portions 1F and 1B, the plurality of waist elastic members 24 and 34 are arranged side-by-side in the vertical direction with a space and are fixed with an adhesive or the like between the skin-side sheets 21 and 31 and the non-skin-side sheets 22 and 32 in a state of being stretched in the lateral direction. Therefore, the front exterior member 20 and the back exterior member 30 fit around the waist of the wearer.

Further, as illustrated in FIG. 2, the front exterior member 20 has a rectangular shape. On the other hand, the back exterior member 30 has an extension portion 30E that extends downward from the back waist portion 1B having a rectangular shape. The extension portion 30E has a substantially trapezoidal shape in which the lateral length thereof decreases downward.

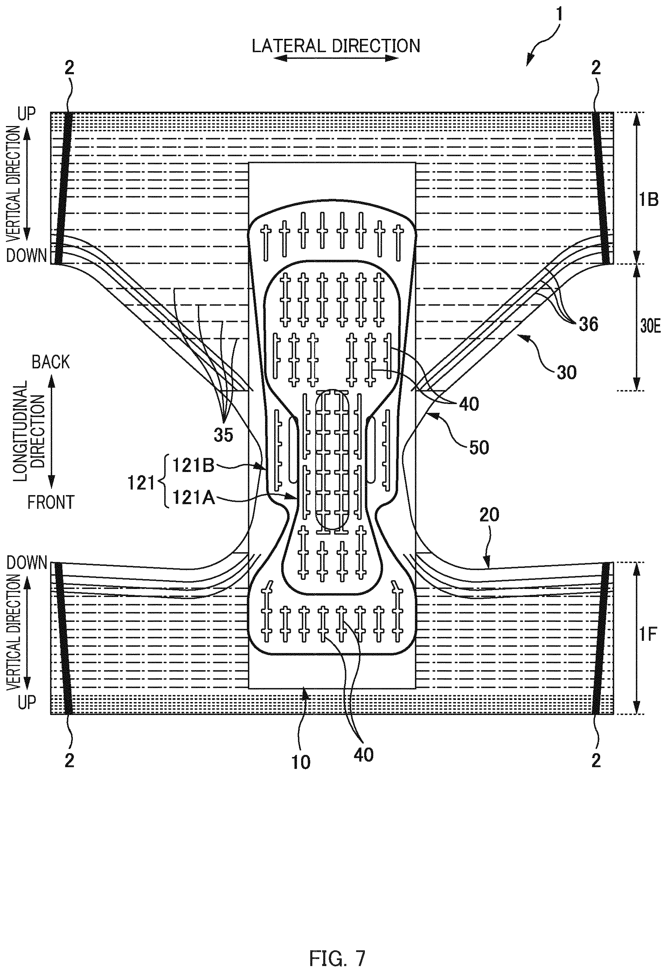

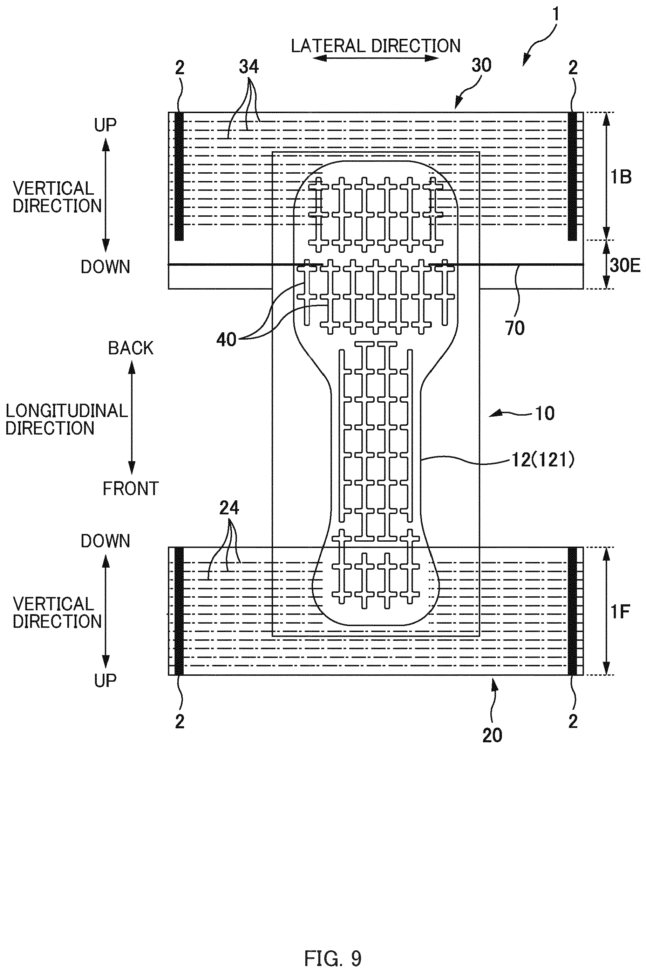

While the basic configuration of the diaper 1 has been described above, the configuration of the diaper 1 is not limited to the above. For example, the diaper 1 may be a two-piece-type diaper, That is, the front exterior member 20 and the back exterior member 30 may be constituted by a single continuous member, or a crotch exterior member that connects the front exterior member 20 and the back exterior member 30 may be provided (see FIG. 7 to be described later). Further, the shape of the extension portion (30E) that extends downward from the waist portion (in this example, the back waist portion 1B) is not limited to a substantially trapezoidal shape, and may be, for example, a rectangular shape (see FIG. 9 to be described later).

### Low-Basis-Weight Region 40 of Absorbent Core 12

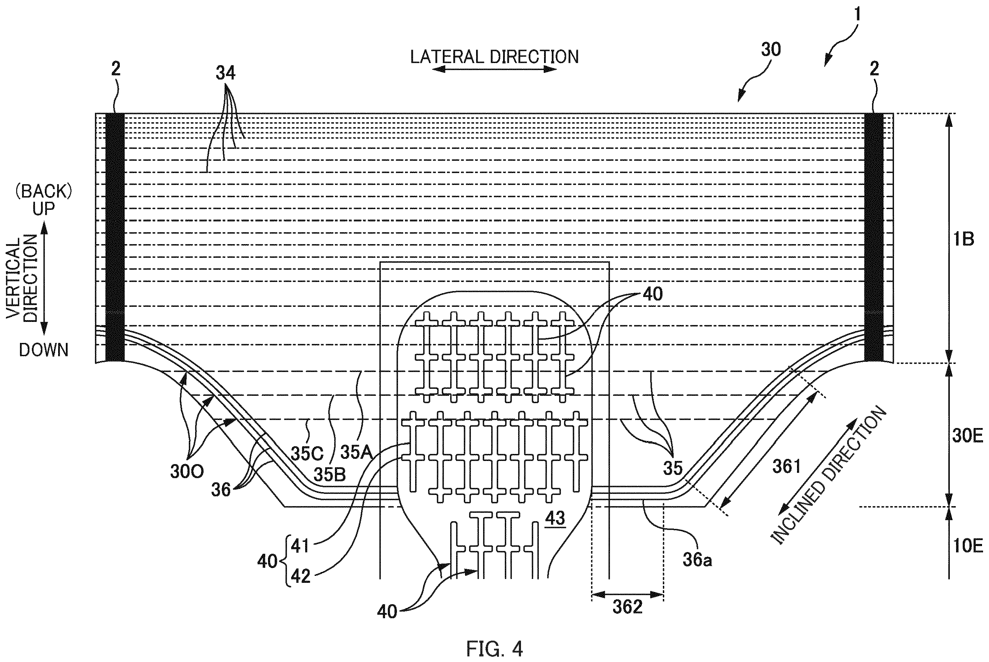

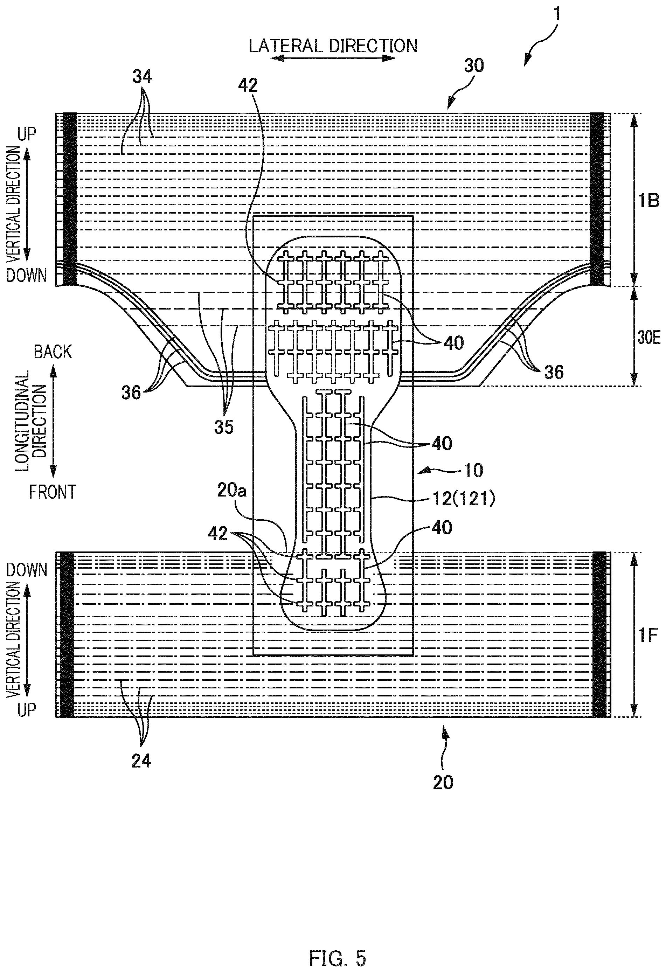

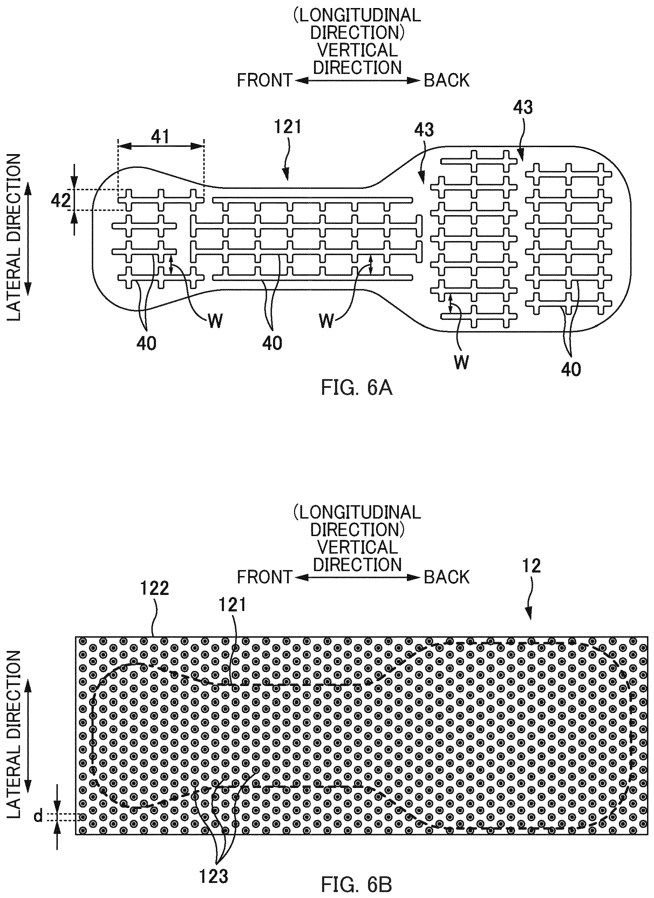

FIGS. 4 and 5 are explanatory diagrams illustrating the low-basis-weight region 40 of the absorbent core 12. FIG. 4 is a schematic plan view of the back exterior member 30 in the unfolded and stretched state. FIG. 5 is a schematic plan view of the diaper 1 in the unfolded and stretched state. FIGS. 6A and 6B are explanatory diagrams illustrating the absorbent core 12. FIG. 7 is an explanatory diagram illustrating a diaper 1 of a modified example and is a schematic plan view of the diaper 1 in the unfolded and stretched state.

The back exterior member 30 that constitutes the diaper 1 has the extension portion 30E that extends downward from the back waist portion 1B. The extension portion 30E can cover the wearer's buttocks. Further, the extension portion 30E has lateral elastic members 35 that stretch and contract in the lateral direction of the diaper 1, and inclined elastic members 36 that stretch and contract in a direction inclined with respect to the lateral direction. The lateral elastic members 35 and the inclined elastic members 36 are string-like elastic members made of rubber, spandex, and the like.

The lateral elastic members are fixed with an adhesive or the like between the skin-side sheet 31 and the non-skin-side sheet 32 that constitute the back exterior member 30, in a state of being stretched in the lateral direction. Therefore, in the stretched state of the diaper 1 (FIG. 4), the lateral elastic members 35 are provided in the extension portion 30E, extending along the lateral direction. In addition, the extension portion 30E is provided with three lateral elastic members 35 arranged side-by-side in the vertical direction with a space. However, the number of the lateral elastic members 35 may be one or plural other than three.

The inclined elastic members 36 are provided extending along the outer edge of the extension portion 30E that has a substantially trapezoidal shape. Therefore, the inclined elastic members 36 each have inclined portions 361 that extend along a direction inclined downward and laterally inward (hereinafter, also referred to as an "inclined direction") and a horizontal portion 362 that extends in the lateral direction. The inclined portion 361 is fixed with an adhesive or the like between the skin-side sheet 31 and the non-skin-side sheet 32, in a state of being stretched in the inclined direction. The horizontal portion 362 is fixed with an adhesive or the like between the skin-side sheet 31 and the non-skin-side sheet 32, in a state of being stretched in the lateral direction. Therefore, in the stretched state of the diaper 1 (FIG. 4), the inclined portions 361 are located in the extension portion 30E with extending along the inclined direction, and the horizontal portion 362 is located in the extension portion 30E with extending along the lateral direction. Further, the extension portion 30E is provided with three inclined elastic members 36 arranged side-by-side in a direction orthogonal to the stretching direction with a space. However, the number of the inclined elastic members 36 may be one or a plural other than three. Further, the inclined elastic member 36 need not have the horizontal portion 362 (see, for example, FIG. 7).

The extension portion 30E is brought into close contact with the wearer's buttocks due to the lateral elastic members 35 and the inclined elastic members 36, and this makes it possible to suppress the positional deviation and the turn-up of the extension portion 30E. In particular, the inclined elastic members 36 are provided extending along the outer edge of the extension portion 30E, and therefore the outer edge portion of the extension portion 30E fits along the contour of the buttocks, making it likely to maintain a state where the extension portion 30E wraps the buttocks. Note that the inclined elastic member 36 is not limited to the string-like elastic member, and may be a sheet-like elastic member such as a stretchable nonwoven fabric or a stretchable film. This can make it less likely to leave the mark of the elastic member along the contour of the buttocks.

Further, as shown in FIG. 6A, the absorbent core 121 has a plurality of low-basis-weight regions 40 in which the basis weight of the absorbent core 121 is lower than that of the surrounding region and which extend along at least either one direction of the vertical direction and the lateral direction. The basis weight of the absorbent core 121 is the mass per unit area (g/m²) of the absorbent core 121 (e.g., liquid-absorbent fibers containing SAP). Further, a region excluding the low-basis-weight regions 40 in the absorbent core 121 will be referred to as a high-basis-weight region 43.

The low-basis-weight region 40 may be a grooved region in which the absorbent core 121 is recessed in the thickness direction, or may be a hole (slit) that penetrates the absorbent core in the thickness direction, or may be a region that is not recessed in the thickness direction and has a lower density than the surrounding region. Comparison of the basis weight of the absorbent core 12 between the low-basis-weight regions 40 and the surrounding region thereof (high-basis-weight region 43) can be performed by a known method. One example is a method of visual comparison. In another method, target portions are cut out from the absorbent core 121 as a sample, and the mass and area of each sample are measured, calculating the basis weight.

As shown in FIG. 6A, the absorbent core 121 of the present embodiment is provided with a plurality of low-basis-weight regions 40 that extend along the vertical direction and the lateral direction. Specifically, the low-basis-weight region 40 has longitudinally-elongated regions 41 that are longer in the vertical direction than in the lateral direction, and laterally-elongated regions 42 that are longer in the lateral direction than in the vertical direction. The vertical length of the longitudinally-elongated region 41 is longer than the lateral length of the laterally-elongated region 42. With respect to one longitudinally-elongated region 41, a plurality of laterally-elongated regions 42 are arranged side-by-side in the vertical direction with a space. Further, the laterally-elongated region 42 intersects the longitudinally-elongated region 41 or extends from a side end of the longitudinally-elongated region 41 toward one side in the lateral direction. In the absorbent core 12, the plurality of low-basis-weight regions 40 are arranged side-by-side in the vertical direction with a space and also side-by-side in the lateral direction with a space.

Due to the difference in stiffness between the low-basis-weight region 40 and the surrounding region, the absorbent core 121 is likely to bend from the low-basis-weight region 40. That is, the absorbent core 121 is likely to bend in the lateral direction along the longitudinally-elongated region 41 that is elongated in the vertical direction, and is likely to bend in the longitudinal direction (vertical direction) along the laterally-elongated region 42. Therefore, the absorbent core 121 is likely to bend along the wearer's body, making the fit of the absorbent core 121 to the wearer good.

Note that the laterally-elongated regions 42 arranged side-by-side in the lateral direction are not continuous but are arranged in the lateral direction with a space. Therefore, it is preferable that the vertical positions of the laterally-elongated regions 42 arranged side-by-side in the lateral direction are aligned. This makes the absorbent core 121 likely to bend in the longitudinal direction (vertical direction) along the laterally-elongated region 42.

Further, providing the low-basis-weight regions 40 in the absorbent core 121 allows the excreted fluid absorbed by the absorbent core 121 to be diffused along the low-basis-weight regions 40 in the vertical direction and lateral direction. Therefore, the excreted fluid is absorbed and held across a wide range of the flat surface of the absorbent core 121, and thus the absorption capacity of the absorbent core 121 can be enhanced.

Further, the diaper 1 has the extension portion 30E from the back waist portion 1B downward, and it is preferable that, as shown in FIG. 4 or 5, the plurality of low-basis-weight regions 40 are provided in the portion of the absorbent core 121 that overlaps the extension portion 30E when the diaper 1 is in the unfolded state.

In the extension portion 30E, the lateral elastic members 35 and the inclined elastic members 36 (hereinafter, collectively referred to as "elastic members 35 and 36") are provided. Therefore, in the case where the low-basis-weight region 40 is not provided in the portion of the absorbent core 121 that overlaps the extension portion 30E, there is a risk that irregular deformation of the absorbent core 121 occurs due to the contractive force of the elastic members 35 and 36. For example, when a part of the absorbent core 121 is deformed so as to protrude toward the non-skin side, a gap is formed between the absorbent core and the wearer to cause the leakage of the excreted fluid. Further, it makes it likely to notice through clothing that the diaper 1 is put on. In particular, the extension portion 30E is positioned on the crotch side compared with the waist portions 1F and 1B, and therefore the proportion (the vertical length) of a region that overlaps the absorbent core 121 becomes large. That is, the elastic members 35 and 36 provided in the extension portion 30E are more likely to affect the contraction of the absorbent core 121 than the waist elastic members 24 and 34 are.

Therefore, the low-basis-weight regions 40 are provided in the portion of the absorbent core 121 that overlaps the extension portion 30E. This makes the low-basis-weight regions 40 of the absorbent core 121 likely to contract due to the elastic members 35 and 36. That is, the low-basis-weight regions 40 are likely to contract in the lateral direction due to the lateral elastic members 35, and are likely to contract in the vertical direction due to the inclined elastic members 36 (inclined portions 361). In particular, the low-basis-weight region s 40 are likely to contract in the lateral direction due to the lateral elastic members 35 that are close to the absorbent core 121. Further, a plurality of low-basis-weight regions 40 (longitudinally-elongated regions 41) are arranged side-by-side in the lateral direction, and a plurality of low-basis-weight regions 40 (laterally-elongated regions 42) are arranged side-by-side in the vertical direction. Therefore, not only a part of the absorbent core 121 that overlaps the extension portion 30E contracts, but also the absorbent core 121 is made likely to regularly contract in the lateral direction and the vertical direction across a wide range in the planar direction. Therefore, in a natural state (non-stretched state) of the diaper 1 before the diaper is put on, it is possible to prevent unintended creases from being formed in the absorbent core 121 due to the elastic members 35 and 36. Further, even while the diaper 1 is put on, it is possible to suppress irregular deformation of the absorbent core 121 (e.g., deformation that the absorbent core protrudes toward the non-skin side) caused by the elastic members 35 and 36, making a gap less likely to be formed between the wearer and the absorbent core 121, and thus enhancing the fit of the absorbent core 121 to the wearer.

Further, when putting on the diaper 1, while expanding the contracted absorbent core 121 in the lateral direction due to the lateral elastic members 35 and simultaneously expanding it in the vertical direction due to the inclined portions 361 of the inclined elastic members 36, the absorbent core 121 (low-basis-weight region 40) can be brought into close contact with the wearer's buttocks. Further, when pulling up the diaper 1 to put it on, the inclined elastic members 36 first stretch, and this makes the lateral elastic members 35 likely to stretch in conjunction therewith. In particular, the inclined elastic members 36 do not extend along the vertical direction, but are inclined upward and laterally outward. Therefore, the lateral elastic members 35 are also likely to stretch in the lateral direction in conjunction with the stretching of the inclined elastic members 36. Accordingly, the contracted absorbent core 121 (low-basis-weight region 40) can be securely expanded in the lateral direction and brought into close contact with the wearer.

Further, providing the low-basis-weight regions 40 in the absorbent core 121 allows the elastic members 35 and 36 to securely contract in the natural state of the diaper 1 before the diaper is put on. Therefore, compared with the case where the low-basis-weight region 40 is not provided in the absorbent core 121 and the elastic members 35 and 36 are slightly stretched, the elastic members 35 and 36 can stretch with a small force, and the diaper 1 can be easily put on.

Further, the lateral elastic members 35 and the inclined elastic members 36 are string-like elastic members. Therefore, compared with the case where, unlike the present embodiment, the entire absorbent core 121 contracts because of providing in the extension portion 30E a stretchable sheet that contracts in the lateral direction and the vertical direction, the absorbent core 121 is likely to contract in a predetermined direction (particularly, in the lateral direction), making irregular deformation of the absorbent core 121 further less likely to occur.

Further, the vertical positions of the lateral elastic members 35 and the inclined elastic members 36 may be deviated from the vertical positions of the low-basis-weight regions 40. However, it is preferable that the vertical positions of the lateral elastic members 35 and the inclined elastic members 36 overlap the vertical positions of the low-basis-weight regions 40. This makes the low-basis-weight regions 40 likely to contract and to expand due to the elastic members 35 and 36.

Further, in the case where the extension portion 30E has a plurality of (in this example, three) lateral elastic members 35 arranged side-by-side in the vertical direction, it is preferable that, when the diaper 1 is in the unfolded state, the vertical positions of the low-basis-weight regions 40 (longitudinally-elongated regions 41) overlap the vertical positions of two or more lateral elastic members 35. In FIG. 4, all the vertical positions of three lateral elastic members 35A to 35C overlap the vertical positions of the longitudinally-elongated regions 41. This makes the low-basis-weight region 40 likely to contract and to expand due to the lateral elastic members 35. Further, it enables the portion of the absorbent core 121 that overlaps the extension portion 30E to regularly contract across a wide range in the vertical direction.

Note that the overlapping of the vertical positions of the lateral elastic members 35 with the vertical positions of the low-basis-weight regions 40 (longitudinally-elongated regions 41) means that the low-basis-weight regions 40 and the lateral elastic members 35 may or may not overlap each other in the thickness direction. For example, in FIG. 4, the lateral elastic members 35 are arranged so as to cross the low-basis-weight regions 40 in the lateral direction, and the low-basis-weight regions 40 and the lateral elastic members 35 overlap each other in the thickness direction. In this case, the low-basis-weight regions 40 are likely to contract and to expand due to the lateral elastic members 35. However, the configuration is not limited thereto. The following configuration is also acceptable: in a region where the extension portion 30E overlaps the absorbent main body 10 in the thickness direction, there is provided a non-stretchable region of the lateral elastic members 35 (e.g., a region where the lateral elastic members 35 are not present or multiple locations of the lateral elastic members 35 are cut), and the low-basis-weight regions 40 and the lateral elastic members 35 (portions that develops stretchability) do not overlap each other in the thickness direction.

Further, it is preferable that the lateral elastic member 35 and the inclined elastic member 36 are not a continuous single member, but are separate members, and that the lateral elastic member 35 and the inclined elastic member 36 have an overlapping portion 30O where the lateral elastic member and the inclined elastic member partially overlap each other in the front-back direction (thickness direction). This makes the low-basis-weight regions 40 likely to securely contract and to expand in the lateral direction due to the lateral elastic members 35. Further, the lateral elastic members 35 are likely to stretch in the lateral direction from the overlapping portions 30O, in conjunction with the stretching of the inclined elastic members 36. Therefore, the contracted absorbent core 121 (low-basis-weight region 40) can be securely expanded in the lateral direction and brought into close contact with the wearer.

Further, it is desirable that the total value of forces (stretching stresses) for stretching the inclined portions 361 (in this example, three inclined portions 361) of the inclined elastic members 36 of the extension portion 30E by a predetermined length in the inclined direction is greater than the total value of forces (stretching stresses) for stretching the lateral elastic members 35 (in this example, three lateral elastic members 35) of the extension portion 30E by the predetermined length in the lateral direction. This makes the lateral elastic members 35 likely to stretch in the lateral direction in conjunction with the strong stretching force of the inclined elastic member 36. Therefore, the contracted absorbent core 121 (low-basis-weight region 40) can be securely expanded in the lateral direction and brought into close contact with the wearer. The stretching stress of each of the elastic members 35 and 36 may be adjusted by adjusting the thickness of the elastic members 35 and 36 and the stretch factor when fixing the elastic members 35 and 36 to the sheet. However, the configuration is not limited to the above configuration, and the total value of the stretching stresses of the lateral elastic members 35 may be equal to or greater than the total value of the stretching stresses of the inclined elastic members 36 (inclined portions 361).

Measuring of the stretching stress of each of the elastic members 35 and 36 can be performed by the following method.
(1) First, for each of the elastic members 35 and 36, a portion where the elastic member is fixed to the sheet with being stretched in the lateral direction or in the inclined direction is cut out from the diaper 1 while the elastic member being in a relaxed state (non-stretched state), preparing a sample for each of the elastic members 35 and 36. However, in the case where it is difficult to prepare or measure samples of each of the elastic members 35 and 36, samples of a set of the elastic members 35 or the elastic members 36 may be prepared and measured.
(2) Next, for each sample, its two end portions corresponding to the lateral direction or the inclined direction of the diaper 1 are held by the chucks of a tensile tester. The chuck-to-chuck distance is adjusted so that the sample is in a state where the sample is not loosened and stretched. Then, the chuck-to-chuck distance is expanded by the tensile tester at a predetermined speed, and the load at a time when the sample has stretched by a predetermined length is acquired. The load at this time is defined as the stretching stress (unit: N) of each of the elastic members 35 and 36. Then, the total value of the stretching stresses of the lateral elastic members 35 and the total value of the stretching stresses of the inclined elastic members 36 are calculated and compared. For example, the chuck-to-chuck distance is set to 50 mm, the predetermined speed (tensile speed) is set to 100 mm/min, and the predetermined length (tensile length) is set to 75 mm. That is, it is recommended to acquire the stress when one elastic member 35 or 36 is stretched by 50% be acquired, and to calculate and compare the total value of the stresses. However, it is recommended to appropriately change the chuck-to-chuck distance, the predetermined speed, and the predetermined length, depending on the samples. For example, it is recommended that the chuck-to-chuck distance is made narrower than the length of the stretched sample which is interposed between the chucks, so that it prevents breakage of the sheets formed of nonwoven fabric or the like interposing the elastic members 35 and 36. Further, in the case where the stretch factors of the elastic members 35 and 36 are low, the stretch factor at the time of acquiring the stretching stress may be lowered by decreasing the predetermined length or the like.

Further, a force (stretching stress) for stretching a region having a unit width in a direction orthogonal to the stretching direction by a predetermined length in the stretching direction may be greater in the region where the inclined elastic members 36 (inclined portions 361) are provided than a region where the lateral elastic members 35 are provided. Therefore, it is recommended to adjust the stretching stress of each of the elastic members 35 and 36 or to adjust the number of elastic members 35 and 36 to be arranged in the region having a unit width. Even in this case, the lateral elastic members 35 are likely to stretch in the lateral direction in conjunction with the strong stretching force of the region where the inclined elastic members 36 are provided.

The stretching stress per unit width in the direction orthogonal to the stretching direction can be measured using a tensile tester in the same manner as described above.
(1) First, for all (in this example, three) of the lateral elastic members 35, a portion where the elastic members are fixed to the sheet in a state of being stretched in the lateral direction is cut out from the diaper 1 while the elastic members being in a relaxed state (non-stretched state), preparing a sample of the region where the lateral elastic members 35 are provided. In addition, for all (three in this case) of the inclined elastic members 36, a portion where the inclined portions 361 of the elastic members 36 are fixed to the sheet in a state of being stretched in the inclined direction is cut out from the diaper 1 while the elastic members 36 being in a relaxed state (non-stretched state), preparing a sample of the region where the inclined elastic members 36 are provided.
(2) Next, the width (the length in the direction orthogonal to the stretching direction) of each sample is measured.
(3) Next, for each sample, its two end portions are held by the chucks of the tensile tester. Then, the chuck-to-chuck distance is expanded at a predetermined speed, and a load at a time when the sample has stretched by a predetermined length is acquired. Based on the load and the width of the sample measured in the step (2), the load is converted into a load per unit width, and the converted load is determined as the stretching stress per unit width of the region where the lateral elastic member 35 or the inclined elastic member 36 is provided.

Note that, in the case where the inclined portions 361 of the inclined elastic members 36 in FIG. 4 are spaced apart from each other in the lateral direction and a plurality of samples are prepared, the average value of the measured values (loads) of each sample may be used.

Further, the extension portion 30E has the plurality of (in this example, three) lateral elastic members 35 arranged side-by-side in the vertical direction. In this case, it is preferable that the lower ends 36a (more preferably, the lower ends of the inclined portions 361) of the inclined elastic members 36 are located below the lowest lateral elastic member 35C. This makes all of the lateral elastic members 35A to 35C likely to stretch in the lateral direction in conjunction with the stretching of the inclined elastic members 36. Therefore, the contracted absorbent core 121 (low-basis-weight region 40) can be securely expanded in the lateral direction and brought into close contact with the wearer.

In addition, in the diaper 1 of the present embodiment, the back exterior member 30 has the extension portion 30E, but the front exterior member 20 does not have the extension portion and has a rectangular shape in a stretched state. Therefore, the rounded buttocks can be covered with the extension portion 30E. Further, although the buttocks are likely to deform due to the movement of the wearer, the lateral elastic members 35 and the inclined elastic members 36 are provided in the extension portion 30E, and the low-basis-weight regions 40 are provided in the portion of the absorbent core 121 that overlaps the extension portion 30E. Therefore, the fit of the extension portion 30E and the absorbent core 121 to the buttocks is maintained. Further, since the front exterior member 20 does not have an extension portion, it is possible to prevent the movement of the legs from hindering during walking or the like due to the extension portion located on the front side.

However, the configuration is not limited to the above. A configuration is acceptable in which both the front exterior member 20 and the back exterior member 30 have extension portions. In this case, it is sufficient that the elastic members 35 and 36 are provided in at least either one of the extension portions, and that the low-basis-weight regions 40 are provided in the portion of the absorbent core 121 that overlaps the extension portion. Further, only the front exterior member 20 may have the extension portion.

Further, the diaper 1 of the present embodiment has the exposed portion 10E of the absorbent main body 10 below the front exterior member 20 and the back exterior member 30. In this case, it is preferable that, the plurality of low-basis-weight regions 40 are also provided in the portion of the absorbent core 12 that is located in the exposed portion 10E when the diaper 1 is in the unfolded state. This makes the portion of the absorbent core 121 that is located in the exposed portion 10E likely to bend due to the low-basis-weight region 40. In particular, in the exposed portion 10E, the bending of the absorbent core 121 is not hindered by the exterior members 20 and 30. Accordingly, the absorbent core 121 is likely to bend along the crotch of the wearer. Therefore, the fit of the portion of the absorbent core 121 that directly receives the excreted fluid to the wearer is enhanced, and the leakage of the excreted fluid can be suppressed.

Further, it is preferable that the low-basis-weight region 40 is provided vertically straddling the lower end of at least either one of the front exterior member 20 and the back exterior member 30 (the lower end 20a of the front exterior member 20 in FIG. 5). In such a case, at the boundary portion between the portion of the absorbent core 121 that is covered with the exterior members 20 and 30 and the portion of the absorbent core 121 that is not covered with the exterior members 20 and 30 and is likely to bend, it is possible to suppress abrupt changes in bending of the absorbent core 121, making the bending possible to be moderate.

Further, it is preferable that, the plurality of low-basis-weight regions 40 are also provided in the portions of the absorbent core 121 that overlap the waist portions 1F and 1B when the diaper 1 is in the unfolded state. This makes the absorbent core 121 likely to bend along the wearer's waist, enhancing the fit of the absorbent core 121 to the wearer.

In addition, the waist portions 1F and 1B are provided with the waist elastic members 24 and 34 that stretch and contract in the lateral direction. Therefore, the low-basis-weight regions 40 are likely to contract due to the waist elastic members 24 and 34, and it is possible to suppress irregular deformation of the portions of the absorbent core 121 that overlap the waist portions 1F and 1B. Further, while expanding the contracted absorbent core 121 (low-basis-weight region 40) in the lateral direction due to the waist elastic members 24 and 34, it allows the absorbent core 121 to come into close contact with the wearer's waist.

Further, it is desirable that, when the diaper 1 is in the unfolded and stretched state, the total area of the low-basis-weight regions 40 located in the portion of the absorbent core 121 that overlaps the extension portion 30E is greater than the total area of the low-basis-weight regions 40 located in the portion of the absorbent core 121 that overlaps the one-side waist portion in the front-back direction (that is, the front waist portion 1F or the back waist portion 1B). In such a case, the portions of the absorbent core 121 that come into contact with the lower abdomen and the buttocks, which are more rounded than the waist, are likely to bend, and the fit of the absorbent core 121 to the wearer is enhanced.

In addition, in the diaper 1 shown in FIG. 4, a portion of the absorbent core 121 that overlap the back waist portion 1B is provided with six low-basis-weight regions 40 arranged side-by-side in the lateral direction. On the other hand, the portion of the absorbent core 121 that overlaps the extension portion 30E is provided with seven low-basis-weight regions 40 arranged side-by-side in the lateral direction. Therefore, the ratio of the area of the low-basis-weight regions 40 per unit area is larger in the portion of the absorbent core 121 that overlaps the extension portion 30E than in the portion of the absorbent core 121 that overlaps the back waist portion 1B. In this case, the fit of the portion of the absorbent core 121 that overlaps the extension portion 30E to the wearer is further enhanced.

Further, it is preferable that at least one laterally-elongated region 42 of the low-basis-weight region 40 is provided in a portion of the absorbent core 121 that overlaps the back waist portion 1B when the diaper 1 is in the unfolded state, and is more preferable that a plurality of (two in FIG. 4) laterally-elongated regions 42 are provided side-by-side in the vertical direction in the portion. This makes the absorbent core 121 likely to bend in the vertical direction (longitudinal direction) along the vertical outline of the wearer's body (from the waist to the rounded buttocks). Therefore, the fit of the absorbent core 121 to the wearer is enhanced.

Similarly, it is preferable that at least one laterally-elongated region 42 of the low-basis-weight region 40 is provided in a portion of the absorbent core 121 that overlaps the front waist portion 1F when the diaper 1 is in the unfolded state, and is more preferable that a plurality of (three in FIG. 5) laterally-elongated regions 42 are provided side-by-side in the vertical direction. This makes the absorbent core 121 likely to bend in the vertical direction (longitudinal direction) along the vertical outline of the wearer's body (from the waist to the rounded lower abdomen). Therefore, the fit of the absorbent core 121 to the wearer is enhanced.

However, the configuration is not limited to the above, and it is sufficient that the low-basis-weight regions 40 are provided at least in the portion of the absorbent core 121 that overlaps the extension portion 30E.

Further, the shape of the low-basis-weight regions 40 shown in FIG. 6A is an example and is not limited thereto. For example, the low-basis-weight regions 40 may have either one of the longitudinally-elongated region 41 and the laterally-elongated region 42. Alternatively, the longitudinally-elongated region 41 and the laterally-elongated region 42 may be provided spaced apart from each other without overlapping. However, if the low-basis-weight regions 40 have both the longitudinally-elongated region 41 and the laterally-elongated region 42, this makes the absorbent core 121 likely to bend in the lateral direction and the vertical direction. Further, the excreted fluid is likely to diffuse in the lateral direction and the vertical direction.

Further, if the longitudinally-elongated region 41 and the laterally-elongated region 42 are arranged intersecting each other, this makes the absorbent core 121 likely to bend in various directions from the intersection portion. This makes the absorbent core 121 likely to bend along the wearer's body.

Further, as shown in FIG. 6A, it is preferable that the low-basis-weight regions 40 are arranged in the absorbent core 121 symmetrically in the lateral direction. This makes it possible to, evenly in the lateral direction, to contract the absorbent core 121 (low-basis-weight region 40) or to diffuse the excreted fluid.

Further, the plurality of low-basis-weight regions 40 provided in the absorbent core 121 are not continuous. Specifically, the plurality of longitudinally-elongated regions 41 arranged side-by-side in the vertical direction are arranged separated in the vertical direction, and therebetween there are provided the high-basis-weight regions 43 extending in the lateral direction. Further, the plurality of laterally-elongated regions 42 arranged side-by-side in the lateral direction are arranged separated in the lateral direction, and therebetween there are provided the high-basis-weight regions 43 extending in the vertical direction. Accordingly, an appropriate stiffness of the absorbent core 121 is maintained, and it is possible to suppress the absorbent core 121 from losing its shape. Further, even when a large amount of excreted fluid is excreted, it is possible to absorb and hold the excreted fluid in the high-basis-weight regions 43 while diffusing the excreted fluid in the low-basis-weight regions 40. Accordingly, the leakage of the excreted fluid can be suppressed.

Further, in the longitudinal back end portion of the absorbent core 121, there is provided the high-basis-weight region 43 that is continuous in the lateral direction between the one-side end and the other-side end of the absorbent core 121. Therefore, the back end portion of the absorbent core 121 is likely to bend in the longitudinal direction due to the difference in stiffness between the high-basis-weight region 43 that is continuous in the lateral direction and the surrounding region. Therefore, the absorbent core 121 is likely to bend in the longitudinal direction along the vertical outline of the wearer's body (from the waist to the rounded buttocks).

Further, since the diaper 1 is provided for adults, it is preferable that the basis weight of the absorbent body 12 including the absorbent core 121 is 400 g/m² or more and the absorption capacity is high. However, in the case where the basis weight of the absorbent body 12 is high, the stiffness increases and the absorbent body 12 is likely to be stiff. However, since the absorbent body 12 (absorbent core 121) of the present embodiment has the low-basis-weight regions 40, the absorbent body 12 is likely to bend along the wearer's body, making it possible to reduce discomfort during putting on the diaper.

The basis weight of the absorbent body 12 can be measured by a well-known method. For example, there is provided a method in which the absorbent body 12 is separated from the diaper 1 and the mass (g) of the absorbent body 12 and the plane area (m²) of the absorbent body 12 are actually measured. Note that, in the case where the absorbent body 12 has the core-wrapping sheet 122, the total mass of the absorbent core 121 and the core-wrapping sheet 122 is measured, and the plane area of the core-wrapping sheet 122 is measured. In addition, in the absorbent body 12 shown in FIGS. 6A and 6B, the shape and size of the absorbent core 121 and the core-wrapping sheet 122 are different from each other, but may be identical.

Further, the absorbent core 121 has a plurality of compressed portions 123 in which a part of the absorbent core 121 is compressed in the thickness direction. In FIG. 6B, a plurality of small circular compressed portions 123 are provided side-by-side in the vertical direction and the lateral direction, the compressed portions 123 obtained by compressing the absorbent core 121 and the core-wrapping sheet 122. The compressed portions 123 make it possible to suppress the absorbent core 121 from losing its shape, and the like. In the compressed portions 123, the absorbent core 121 has a smaller thickness (is a recessed portion) than in the surrounding region, and the density of the liquid-absorbent fibers that constitute the absorbent core 121 is high.

Further, as shown in FIG. 6A, in the absorbent core 121, a plurality of longitudinally-elongated regions 41 (low-basis-weight regions 40) are arranged side-by-side in the lateral direction with a space. In this case, it is preferable that in the stretched state of the diaper 1, the average length d (diameter) of the compressed portions 123 in the lateral direction is smaller than an average space W between the longitudinally-elongated regions 42 arranged side-by-side in the lateral direction.

This increases the probability that the compressed portions 123 are placed in the high-basis-weight regions 43 that are located between the longitudinally-elongated regions 41. It also decreases the probability that the compressed portions 123 are placed at the boundary portion between the longitudinally-elongated region 41 (low-basis-weight region 40) and the high-basis-weight region 43. Therefore, it is possible to suppress he absorbent core 121 from becoming less likely to contract due to integrating of the longitudinally-elongated regions 41 with the high-basis-weight regions 43 by the compressed portions 123 that are positioned at the boundary portion. This allows the absorbent core 121 (the longitudinally-elongated regions 41) to regularly contracted by the elastic members 35 and 36.

Further, as in the diaper 1 of the modified example shown in FIG. 7, a configuration is acceptable in which the absorbent core 121 has a two-layer structure constituted by an upper core 121A and a lower core 121B that is arranged on the non-skin side in the thickness direction with respect to the upper core 121A. In such a case, the absorption capacity of the diaper 1 is enhanced. Even in this case, it is preferable that the lateral elastic members 35 and the inclined elastic members 36 are provided in extension portions 30E that extend downward from the waist portions 1F and 1B (the back waist portion 1B in FIG. 7). Further, it is preferable that the low-basis-weight regions 40 are provided in the portion of the absorbent core 121 that overlaps the extension portion 30E. This makes it possible to contract the low-basis-weight regions 40 by the elastic members 35 and 36, making it possible to suppress irregular deformation of the absorbent core 121. Further, the contracted absorbent core 121 (low-basis-weight region 40) can be expanded and brought into close contact with the wearer.

In addition, in the absorbent core 121 having a two-layer structure, stiffness further increases and the absorbent core is more likely to be stiff. However, the low-basis-weight regions 40 makes the absorbent core 121 likely to bend, making it possible to reduce discomfort during putting on the diaper. Further, in FIG. 7, the low-basis-weight regions 40 are provided in both a portion of the upper core 121A that overlaps the extension portion 30E and a portion of the lower core 121B that overlap the extension portion 30E. However, it is sufficient that the low-basis-weight regions 40 are provided in at least either one of the upper core 121A and the lower core 121B.

Further, the diaper 1 in FIG. 7 is a two-piece type which has no exposed portion 10E of the absorbent main body 10, and includes a crotch exterior member 50 that is located the front exterior member 20 and the back exterior member 30 and connects them. In this case, the extension portion on one side in the front-back direction is a region extending from the lower ends of the joining portions 2 to the lower end of the exterior member on the one side. Further, although not shown, in the case of a two-piece-type diaper 1 having an exterior member in which the front exterior member 20 and the back exterior member 30 are seamlessly continuous with each other, the extension portion is a region from the lower ends of the joining portions 2 to the lower end of the diaper 1.

### Second Embodiment

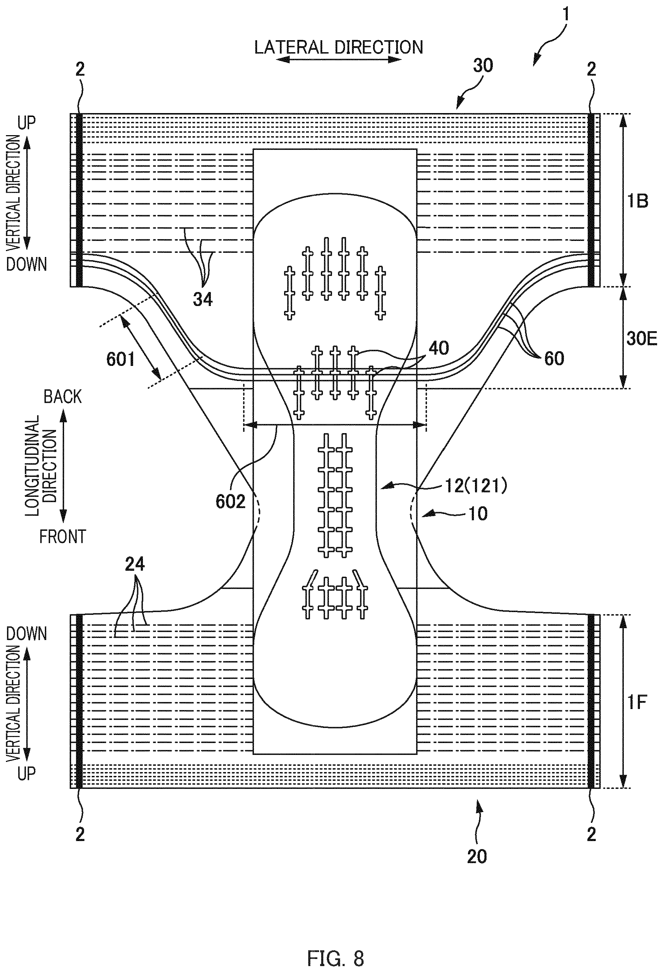

FIG. 8 is an explanatory diagram illustrating the diaper 1 of a second embodiment, and is a schematic plan view of the diaper 1 in the unfolded and stretched state. The lateral elastic members and the inclined elastic members provided in the extension portion 30E are not limited to being separate members, and may be a single member.

Similar to the inclined elastic members 36 of the first embodiment, elastic members 60 shown in FIG. 8 are provided along the outer edge of the extension portion 30E having a substantially trapezoidal shape. Therefore, each of the elastic members 60 is fixed to the sheet in a state of being stretched in the lateral direction. And the elastic member 60 includes: a horizontal portion 602 that stretches and contracts in the lateral direction (corresponding to the lateral elastic member); and inclined portions 601 that are fixed to the sheet in a state of being stretched in the inclined direction and stretches and contracts in the inclined direction. Even in this case, the low-basis-weight regions 40 provided in the portion of the absorbent core 121 that overlaps the extension portion 30E can be contracted in the lateral direction and the vertical direction due to the inclined portions 601 and the horizontal portion 602, making it possible to suppress irregular deformation of the absorbent core 121. Further, while expanding the contracted absorbent core 121 in the lateral direction and the vertical direction due to the inclined portions 601 and the horizontal portion 602, it allows the absorbent core 121 to come into close contact with the wearer's body.

### Third Embodiment

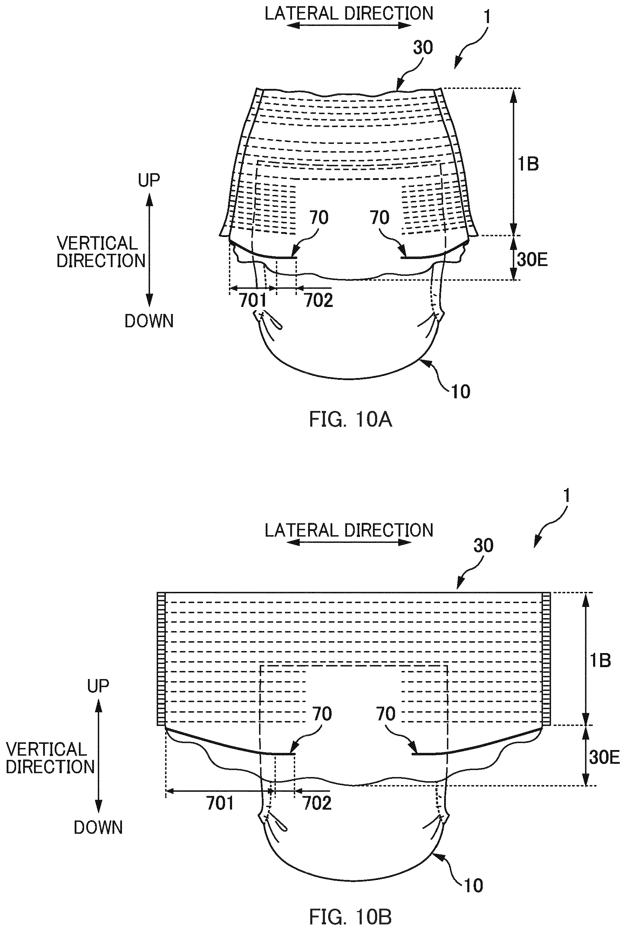

FIG. 9 is an explanatory diagram illustrating a diaper 1 of a third embodiment and is a schematic plan view of the diaper 1 in the unfolded and stretched state. FIG. 10A is a schematic front view of the diaper 1 of the third embodiment in the natural state, and FIG. 10B is a schematic front view of the diaper 1 of the third embodiment in the put-on state.

In the diapers 1 of the first embodiment and the second embodiment, all or some of the elastic members fixed to the sheet in a state of being stretched along the lateral direction are defined as the lateral elastic members 35 and 602, and all or some of the elastic members fixed to the sheet in a state of being stretched along the inclined direction are defined as the inclined elastic members 36 and 601. Therefore, in a state where the diaper 1 is stretched to an extent that wrinkles are eliminated, the lateral elastic members 35 and 602 are arranged along the lateral direction, and the inclined elastic members 36 (inclined portions 361) and 601 are arranged along the inclined direction.

In the diaper 1 of the third embodiment, the extension portion 30E is provided with elastic members 70 fixed to the sheet in a state of being stretched along the lateral direction, but the extension portion 30E is not provided with an elastic member fixed to the sheet in a state of being stretched along the inclined direction. Even in this case, in the natural state (non-stretched state) of the diaper 1 shown in FIG. 10A and in the put-on state of the diaper 1 shown in FIG. 10B, the elastic members 70 each have a horizontal portion 702 that extends along the lateral direction and that stretches and contracts in the lateral direction (corresponding to the lateral elastic member), and an inclined portion 701 that extends along the inclined direction and that stretches and contracts in the inclined direction (corresponding to the inclined elastic member) .

Therefore, the low-basis-weight regions 40 provided in the portion of the absorbent core 121 that overlaps the extension portion 30E can be contracted in the lateral direction and the vertical direction due to the inclined portions 701 and the horizontal portion 702, making it possible to suppress irregular deformation of the absorbent core 121. Further, while expanding the contracted absorbent core 121 in the lateral direction and the vertical direction due to the inclined portions 701 and the horizontal portions 702, it allows the absorbent core 121 to come into close contact with the wearer's body.

### REFERENCE SIGNS LIST

1: diaper (underpants-shaped absorbent article), 2: joining portion,
1F: front waist portion, 1B: back waist portion, 1C: crotch portion,
10: absorbent main body, 10E: exposed portion,
11: top sheet, 12: absorbent body,
121: absorbent core, 122: core-wrapping sheet, 123: compressed portion,
13: back sheet, 14: exterior sheet,
15: leak-proof wall portion, 151: leak-proof-wall elastic member,
20: front exterior member (exterior member), 21: skin-side sheet,
22: non-skin-side sheet,
23: cover sheet, 24: waist elastic member,
30: back exterior member (exterior member), 30E: extension portion,
31: skin-side sheet, 32: non-skin-side sheet,
33: cover sheet, 34: waist elastic member,
35: lateral elastic member, 36: inclined elastic member,
40: low-basis-weight region, 41: longitudinally-elongated region,
42: laterally-elongated region, 43: high-basis-weight region,
50: crotch exterior member,
60: elastic member,
601: inclined portion (inclined elastic member), 602: horizontal portion (lateral elastic member)
70: elastic member,
701: inclined portion (inclined elastic member), 702: horizontal portion (lateral elastic member)

## Claims

1. An underpants-shaped absorbent article (1) having a vertical direction, a lateral direction, and a front-back direction,
the absorbent article comprising:
an absorbent main body (10) that includes an absorbent core; and
an exterior member (30) that is arranged on a non-skin side with respect to the absorbent main body,
two lateral side portions of each of a pair of waist portions (1F, 1B) in the exterior member being joined by a pair of joining portions (2),
the exterior member (30) having an extension portion,
the extension portion (30E) being a portion that extends downward from at least either one of the pair of waist portions (1F, 1B),
the extension portion (30E) having
a string-like lateral elastic member that stretches and contracts in the lateral direction, and
an inclined elastic member that stretches and contracts in a direction inclined with respect to the lateral direction,
a plurality of low-basis-weight regions (40) being provided in a portion of the absorbent core (12, 121) that overlaps the extension portion when the underpants-shaped absorbent article is in an unfolded state,
the low-basis-weight region (40) being a region in which a basis weight of the absorbent core (12, 121) is lower than in a surrounding region and that extends along at least either one direction of the vertical direction and the lateral direction; and
wherein the low-basis-weight region (40) has a plurality of longitudinally-elongated regions (41) that are longer in the vertical direction than in the lateral direction,
the plurality of longitudinally-elongated regions (41) are arranged side-by-side in the lateral direction with a space,
the absorbent core (12, 121) has a plurality of compressed portions (123) in which a part of the absorbent core (12, 121) is compressed in a thickness direction, and
in a stretched state of the underpants-shaped absorbent article (1),
an average length of the compressed portions (123) in the lateral direction is smaller than an average space between the longitudinally-elongated regions (41) arranged side-by-side in the lateral direction.

2. The underpants-shaped absorbent article according to claim 1, wherein
the lateral elastic member (35) and the inclined elastic member (36)
are separate members and
have an overlapping portion where the lateral elastic member and the inclined elastic member partially overlap each other in the front-back direction.

3. The underpants-shaped absorbent article according to claim 1 or 2, wherein
a total value of a force for stretching the inclined elastic member (36) of the extension portion (30E) by a predetermined length in the inclined direction
is greater than
a total value of a force for stretching the lateral elastic member (35) of the extension portion (30E) by the predetermined length in the lateral direction.

4. The underpants-shaped absorbent article according to any one of claims 1 to 3, wherein
the exterior member (30) has the extension portion (30E) that extends downward from a back waist portion.

5. The underpants-shaped absorbent article according to any one of claims 1 to 4, wherein
a one-side portion of the exterior member (30) that is located on an one side in the front-back direction has the extension portion (30E),
an other-side portion of the exterior member (30) that is located on another side in the front-back direction has a rectangular shape in a stretched state,
the absorbent main body (10) has an exposed portion (10E) below the one-side portion of the exterior member (30) in the front-back direction and below the other-side portion of the exterior member (30) in the front-back direction,
the exposed portion (10E) having a non-skin-side surface that is exposed to an outside, and
a plurality of low-basis-weight regions (40) are provided in a portion of the absorbent core (12, 121) that is located in the exposed portion (10E) when the underpants-shaped absorbent article is in the unfolded state,
the low-basis-weight region (40) being a region in which the basis weight of the absorbent core is lower than in a surrounding region and which extends along at least either one direction of the vertical direction and the lateral direction.

6. The underpants-shaped absorbent article according to any one of claims 1 to 5, wherein
the extension portion (30E) has a plurality of the lateral elastic members arranged side-by-side in the vertical direction,
the low-basis-weight region (40) has a longitudinally-elongated region (41) that is longer in the vertical direction than in the lateral direction, and
in the unfolded state of the underpants-shaped absorbent article,
a vertical position of the longitudinally-elongated region (41) overlaps vertical positions of two or more of the lateral elastic members.

7. The underpants-shaped absorbent article according to any one of claims 1 to **6,** wherein
the extension portion (30E) has a plurality of the lateral elastic members (35) arranged side-by-side in the vertical direction, and
a lower end of the inclined elastic member (36) is located below the lowest lateral elastic member.

8. The underpants-shaped absorbent article according to any one of claims 1 to **7,** wherein
the low-basis-weight region (40) has
a longitudinally-elongated region (41) that is longer in the vertical direction than in the lateral direction and
a laterally-elongated region (42) that is longer in the lateral direction than in the vertical direction, and
the longitudinally-elongated region (41) and the laterally-elongated region (42) are arranged intersecting each other.

9. The underpants-shaped absorbent article according to any one of claims 1 to **8,** wherein
a basis weight of the absorbent body including the absorbent core is 400 g/m² or more.

10. The underpants-shaped absorbent article according to any one of claims 1 to **9,** wherein
the exterior member (30) has a waist elastic member that stretches and contracts in the lateral direction, in the waist portion, and
a plurality of low-basis-weight regions (40) is provided in a portion of the absorbent core that overlaps the waist portion when the underpants-shaped absorbent article is in the unfolded state,
the low-basis-weight region (40) being a region in which the basis weight of the absorbent core is lower than in a surrounding region and that extends along at least either one direction of the vertical direction and the lateral direction.

11. The underpants-shaped absorbent article according to claim 10, wherein
when the underpants-shaped absorbent article is in the unfolded state and in a stretched state,
a total area of the low-basis-weight regions (40) located in the portion of the absorbent core that overlaps the extension portion (30E)
is larger than
a total area of the low-basis-weight regions (40) located in the portion of the absorbent core that overlaps the waist portion.

12. The underpants-shaped absorbent article according to claim 10 or 11, wherein
the low-basis-weight region (40) has a laterally-elongated region (42) that is longer in the lateral direction than in the vertical direction, and
the laterally-elongated region (42) is located in a portion of the absorbent core that overlaps a back waist portion when the underpants-shaped absorbent article is in the unfolded state.

13. The underpants-shaped absorbent article according to any one of claims 10 to 12, wherein
the low-basis-weight region (40) has a laterally-elongated region (42) that is longer in the lateral direction than in the vertical direction, and
a plurality of the laterally-elongated regions (42) are located side-by-side in the vertical direction in a portion of the absorbent core that overlaps a front waist portion when the underpants-shaped absorbent article is in the unfolded state.

14. The underpants-shaped absorbent article according to any one of claims 1 to 13, wherein
the absorbent main body (10) has an exposed portion below a front portion of the exterior member (30) and below a back portion of the exterior member (30),
the exposed portion (10E) having a non-skin-side surface that is exposed to an outside, and
the low-basis-weight region (40) is provided vertically straddling a lower end of at least either one of the front portion of the exterior member (30) and the back portion of the exterior member.

## Patentansprüche

1. Unterhosen-förmiger absorbierender Artikel (1), der eine vertikale Richtung, eine laterale Richtung und eine Vom-Hinten-Richtung aufweist,
wobei der absorbierende Artikel Folgendes umfasst:
einen absorbierenden Hauptkörper (10), der einen Saugkern einschließt; und
ein äußeres Element (30), das auf einer Nicht-Hautseite in Bezug auf den absorbierenden Hauptkörper angeordnet ist,
wobei zwei laterale Seitenteile von jedem eines Paar von Taillenteilen (1F, 1B) in dem äußeren Element durch ein Paar von Verbindungsteilen (2) verbunden sind,
das äußere Element (30) einen Verlängerungsteil aufweist,
wobei der Verlängerungsteil (30E) ein Teil ist, der sich nach unten von zumindest einem des Paars von Taillenteilen (1F, 1B) erstreckt,
wobei der Verlängerungsteil (30E) Folgendes aufweist:
ein fadenähnliches laterales elastisches Element, das sich in der lateralen Richtung dehnt und zusammenzieht, und
ein schräges elastisches Element, das sich in einer Richtung dehnt und zusammenzieht, die in Bezug auf die laterale Richtung schräg ist,
wobei eine Vielzahl von Bereichen mit geringem Flächengewicht (40) in einem Teil des Saugkerns (12, 121) bereitgestellt ist, der mit dem Verlängerungsteil überlappt, wenn der Unterhosen-förmige absorbierende Artikel in einem entfalteten Zustand vorliegt,
wobei der Bereich mit geringem Flächengewicht (40) ein Bereich ist, in dem ein Flächengewicht des Saugkerns (12, 121) geringer ist als in einem umgebenden Bereich und der sich entlang von zumindest einer Richtung der vertikalen Richtung und der lateralen Richtung erstreckt; und
wobei der Bereich mit geringem Flächengewicht (40) eine Vielzahl von längs langgestreckten Bereichen (41) aufweist, die in der vertikalen Richtung länger sind als in der lateralen Richtung,
wobei die Vielzahl von längs langgestreckten Bereichen (41) in der lateralen Richtung mit einem Abstand nebeneinander angeordnet sind,
der Saugkern (12, 121) eine Vielzahl von komprimierten Teilen (123) aufweist, in denen ein Teil des Saugkerns (12, 121) in einer Dickenrichtung komprimiert ist und
in einem gedehnten Zustand des Unterhosen-förmigen absorbierenden Artikels (1)
eine durchschnittliche Länge der komprimierten Teile (123) in der lateralen Richtung kleiner ist als ein durchschnittlicher Abstand zwischen den längs langgestreckten Bereichen (41), die in der lateralen Richtung nebeneinander angeordnet sind.

2. Unterhosen-förmiger absorbierender Artikel nach Anspruch 1, wobei
das laterale elastische Element (35) und das schräge elastische Element (36)
separate Elemente sind und
einen überlappenden Teil aufweisen, wo das laterale elastische Element und das schräge elastische Element miteinander in der Vorn-Hinten-Richtung teilweise überlappen.

3. Unterhosen-förmiger absorbierender Artikel nach Anspruch 1 oder 2, wobei
ein Gesamtwert einer Kraft zum Dehnen des schrägen elastischen Elements (36) des Verlängerungsteils (30E) um eine vorgegebene Länge in der schrägen Richtung
größer ist als
ein Gesamtwert einer Kraft zum Dehnen des lateralen elastischen Elements (35) des Verlängerungsteils (30E) um die vorgegebene Länge in der lateralen Richtung.

4. Unterhosen-förmiger absorbierender Artikel nach einem der Ansprüche 1 bis 3, wobei
das äußere Element (30) den Verlängerungsteil (30E) aufweist, der sich von einem hinteren Taillenteil nach unten erstreckt.

5. Unterhosen-förmiger absorbierender Artikel nach einem der Ansprüche 1 bis 4, wobei
ein Teil einer Seite des äußeren Elements (30), der sich auf einer Seite in der Vorn-Hinten-Richtung befindet, den Verlängerungsteil (30E) aufweist,
ein Teil der anderen Seite des äußeren Elements (30), der sich auf einer anderen Seite in der Vorn-Hinten-Richtung befindet, eine rechteckige Form in einem gedehnten Zustand aufweist,
der absorbierende Hauptkörper (10) einen exponierten Teil (10E) unterhalb des Teils der einen Seite des äußeren Elements (30) in der Vorn-Hinten-Richtung und unterhalb des anderen Teils der anderen Seite des äußeren Elements (30) in der Vorn-Hinten-Richtung aufweist,
wobei der exponierte Teil (10E) eine Nicht-Hautseitenoberfläche aufweist, die gegenüber einer Außenseite exponiert ist, und
eine Vielzahl von Bereichen mit geringem Flächengewicht (40) in einem Teil des Saugkerns (12, 121) bereitgestellt ist, der sich in dem exponierten Teil (10E) befindet, wenn der Unterhosen-förmige absorbierende Artikel in dem entfalteten Zustand vorliegt,
wobei der Bereich mit geringem Flächengewicht (40) ein Bereich ist, in dem das Flächengewicht des Saugkerns geringer ist als in einem umgebenden Bereich und der sich entlang von zumindest einer Richtung der vertikalen Richtung und der lateralen Richtung erstreckt.

6. Unterhosen-förmiger absorbierender Artikel nach einem der Ansprüche 1 bis 5, wobei
der Verlängerungsteil (30E) eine Vielzahl der lateralen elastischen Elemente aufweist, die in der vertikalen Richtung nebeneinander angeordnet sind,
der Bereich mit geringem Flächengewicht (40) einen längs langgestreckten Bereich (41) aufweist, der in der vertikalen Richtung länger ist als in der lateralen Richtung, und
in dem entfalteten Zustand des Unterhosen-förmigen absorbierenden Artikels
eine vertikale Position des längs langgestreckten Bereichs (41) mit vertikalen Positionen von zwei oder mehr der lateralen elastischen Elemente überlappt.

7. Unterhosen-förmiger absorbierender Artikel nach einem der Ansprüche 1 bis 6, wobei
der Verlängerungsteil (30E) eine Vielzahl der lateralen elastischen Elemente (35) aufweist, die in der vertikalen Richtung nebeneinander angeordnet sind, und
sich ein unteres Ende des schrägen elastischen Elements (36) unterhalb des untersten lateralen elastischen Elements befindet.

8. Unterhosen-förmiger absorbierender Artikel nach einem der Ansprüche 1 bis 7, wobei
der Bereich mit geringem Flächengewicht (40) Folgendes aufweist:
einen längs langgestreckten Bereich (41), der in der vertikalen Richtung länger ist als in der lateralen Richtung, und
einen lateral langgestreckten Bereich (42), der in der lateralen Richtung länger ist als in der vertikalen Richtung, und
der längs langgestreckte Bereich (41) und der lateral langgestreckte Bereich (42) angeordnet sind, dass sie einander schneiden.

9. Unterhosen-förmiger absorbierender Artikel nach einem der Ansprüche 1 bis 8, wobei
ein Flächengewicht des Saugkörpers, der den Saugkern einschließt, 400 g/m² oder mehr beträgt.

10. Unterhosen-förmiger absorbierender Artikel nach einem der Ansprüche 1 bis 9, wobei
das äußere Element (30) ein elastisches Taillenelement aufweist, das sich in dem Taillenteil in der lateralen Richtung dehnt und zusammenzieht, und
eine Vielzahl von Bereichen mit geringem Flächengewicht (40) in einem Teil des Saugkerns bereitgestellt ist, der mit dem Taillenteil überlappt, wenn der Unterhosen-förmige absorbierende Artikel in dem entfalteten Zustand vorliegt,
wobei der Bereich mit geringem Flächengewicht (40) ein Bereich ist, in dem das Flächengewicht des Saugkerns geringer ist als in einem umgebenden Bereich und der sich entlang von zumindest einer Richtung der vertikalen Richtung und der lateralen Richtung erstreckt.

11. Unterhosen-förmiger absorbierender Artikel nach Anspruch 10, wobei,
wenn der Unterhosen-förmige absorbierende Artikel in dem entfalteten Zustand und in einem gedehnten Zustand vorliegt,
eine Gesamtfläche der Bereiche mit geringem Flächengewicht (40), die sich in dem Teil des Saugkerns befinden, der mit dem Verlängerungsteil (30E) überlappt,
größer ist als
eine Gesamtfläche der Bereiche mit geringem Flächengewicht (40), die sich in dem Teil des Saugkerns befinden, der mit dem Taillenteil überlappt.

12. Unterhosen-förmiger absorbierender Artikel nach Anspruch 10 oder 11, wobei
der Bereich mit geringem Flächengewicht (40) einen lateral langgestreckten Bereich (42) aufweist, der in der lateralen Richtung länger ist als in der vertikalen Richtung, und
sich der lateral langgestreckte Bereich (42) in einem Teil des Saugkerns befindet, der mit einem hinteren Taillenteil überlappt, wenn der Unterhosen-förmige absorbierende Artikel in dem entfalteten Zustand vorliegt.

13. Unterhosen-förmiger absorbierender Artikel nach einem der Ansprüche 10 bis 12,
wobei
der Bereich mit geringem Flächengewicht (40) einen lateral langgestreckten Bereich (42) aufweist, der in der lateralen Richtung länger ist als in der vertikalen Richtung, und
sich eine Vielzahl von lateral langgestreckten Bereichen (42) nebeneinander in der vertikalen Richtung in einem Teil des Saugkerns befindet, der mit einem vorderen Taillenteil überlappt, wenn der Unterhosen-förmige absorbierende Artikel in dem entfalteten Zustand vorliegt.

14. Unterhosen-förmiger absorbierender Artikel nach einem der Ansprüche 1 bis 13, wobei
der absorbierende Hauptkörper (10) einen exponierten Teil unterhalb eines vorderen Teils des äußeren Elements (30) und unterhalb eines hinteren Teils des äußeren Elements (30) aufweist,
wobei der exponierte Teil (10E) eine Nicht-Hautseitenoberfläche aufweist, die gegenüber einer Außenseite exponiert ist, und
der Bereich mit geringem Flächengewicht (40) bereitgestellt ist, dass er ein unteres Ende von zumindest einem des vorderen Teils des äußeren Elements (30) und des hinteren Teils des äußeren Elements vertikal überspannt.

## Revendications

1. Article absorbant en forme de culotte (1) ayant une direction verticale, une direction latérale et une direction allant d'avant en arrière,
l'article absorbant comportant :
un corps principal absorbant (10) qui comprend une partie centrale absorbante ; et
un élément extérieur (30) qui est agencé sur un côté non orienté vers la peau par rapport au corps principal absorbant,
deux parties côté latéral de chacune d'une paire de parties au niveau de la taille (1F, 1B) dans l'élément extérieur qui sont assemblées par une paire de parties d'assemblage (2),
l'élément extérieur (30) ayant une partie d'extension,
la partie d'extension (30E) étant une partie qui s'étend vers le bas à partir d'au moins l'une ou l'autre de la paire de parties au niveau de la taille (1F, 1B),
la partie d'extension (30E) ayant
un élément élastique latéral en forme de ficelle qui s'étire et se contracte dans la direction latérale, et
un élément élastique incliné qui s'étire et se contracte dans une direction inclinée par rapport à la direction latérale,
les régions d'une pluralité de régions de faible masse surfacique (40) étant mises en œuvre dans une partie de la partie centrale absorbante (12, 121) qui chevauche la partie d'extension quand l'article absorbant en forme de culotte est dans un état déplié,
la région de faible masse surfacique (40) étant une région dans laquelle une masse surfacique de la partie centrale absorbante (12, 121) est inférieure à celle dans une région environnante et qui s'étend le long d'au moins une direction parmi la direction verticale et la direction latérale ; et
dans lequel la région de faible masse surfacique (40) a une pluralité de régions allongées longitudinalement (41) qui sont plus longues dans la direction verticale que dans la direction latérale,
les régions de la pluralité de régions allongées longitudinalement (41) sont agencées côte à côte dans la direction latérale avec un espace,
la partie centrale absorbante (12, 121) a une pluralité de parties comprimées (123) dans lesquelles une partie de la partie centrale absorbante (12, 121) est comprimée dans une direction allant dans le sens de l'épaisseur, et
dans un état étiré de l'article absorbant en forme de culotte (1),
une longueur moyenne des parties comprimées (123) dans la direction latérale est inférieure à un espace moyen entre les régions allongées longitudinalement (41) agencées côte à côte dans la direction latérale.

2. Article absorbant en forme de culotte selon la revendication 1, dans lequel
l'élément élastique latéral (35) et l'élément élastique incliné (36)
sont des éléments séparés et
ont une partie de chevauchement là où l'élément élastique latéral et l'élément élastique incliné se chevauchent partiellement l'un par rapport à l'autre dans la direction allant d'avant en arrière.

3. Article absorbant en forme de culotte selon la revendication 1 ou la revendication 2, dans lequel
une valeur totale d'une force appliquée pour étirer l'élément élastique incliné (36) de la partie d'extension (30E) sur une longueur prédéterminée dans la direction inclinée
est supérieure à
une valeur totale d'une force appliquée pour étirer l'élément élastique latéral (35) de la partie d'extension (30E) sur la longueur prédéterminée dans la direction latérale.

4. Article absorbant en forme de culotte selon l'une quelconque des revendications 1 à 3, dans lequel
l'élément extérieur (30) a la partie d'extension (30E) qui s'étend vers le bas à partir d'une partie arrière au niveau de la taille.

5. Article absorbant en forme de culotte selon l'une quelconque des revendications 1 à 4, dans lequel
une partie d'un côté de l'élément extérieur (30) qui se trouve sur un dit un côté dans la direction allant d'avant en arrière a la partie d'extension (30E),
une partie d'un autre côté de l'élément extérieur (30) qui se trouve sur un dit un autre côté dans la direction allant d'avant en arrière a une forme rectangulaire dans un état étiré,
le corps principal absorbant (10) a une partie exposée (10E) sous ladite partie d'un côté de l'élément extérieur (30) dans la direction allant d'avant en arrière et sous ladite partie d'un autre côté de l'élément extérieur (30) dans la direction allant d'avant en arrière,
la partie exposée (10E) ayant une surface côté non orienté vers la peau qui est exposée vers l'extérieur, et
les régions d'une pluralité de régions de faible masse surfacique (40) sont mises en œuvre dans une partie de la partie centrale absorbante (12, 121) qui se trouve dans la partie exposée (10E) quand l'article absorbant en forme de culotte est dans l'état déplié,
la région de faible masse surfacique (40) étant une région dans laquelle la masse surfacique de la partie centrale absorbante est inférieure à celle dans une région environnante et qui s'étend le long d'au moins une direction parmi la direction verticale et la direction latérale.

6. Article absorbant en forme de culotte selon l'une quelconque des revendications 1 à 5, dans lequel
la partie d'extension (30E) a une pluralité d'éléments élastiques latéraux agencés côte à côte dans la direction verticale,
la région de faible masse surfacique (40) a une région allongée longitudinalement (41) qui est plus longue dans la direction verticale que dans la direction latérale, et
dans l'état déplié de l'article absorbant en forme de culotte,
une position verticale de la région allongée longitudinalement (41) chevauche les positions verticales de deux éléments ou plus parmi les éléments élastiques latéraux.

7. Article absorbant en forme de culotte selon l'une quelconque des revendications 1 à 6, dans lequel
la partie d'extension (30E) a une pluralité d'éléments élastiques latéraux (35) agencés côte à côte dans la direction verticale, et
une extrémité inférieure de l'élément élastique incliné (36) se trouve sous l'élément élastique latéral le plus bas.

8. Article absorbant en forme de culotte selon l'une quelconque des revendications 1 à 7, dans lequel
la région de faible masse surfacique (40) a
une région allongée longitudinalement (41) qui est plus longue dans la direction verticale que dans la direction latérale et
une région allongée latéralement (42) qui est plus longue dans la direction latérale que dans la direction verticale, et
la région allongée longitudinalement (41) et la région allongée latéralement (42) sont agencées de manière à se croiser l'une l'autre.

9. Article absorbant en forme de culotte selon l'une quelconque des revendications 1 à 8, dans lequel
une masse surfacique du corps absorbant comprenant la partie centrale absorbante est de 400 g/m² ou plus.

10. Article absorbant en forme de culotte selon l'une quelconque des revendications 1 à 9, dans lequel
l'élément extérieur (30) a un élément élastique au niveau de la taille qui s'étire et se contracte dans la direction latérale, dans la partie au niveau de la taille, et
une pluralité de régions de faible masse surfacique (40) est mise en œuvre dans une partie de la partie centrale absorbante qui chevauche la partie au niveau de la taille quand l'article absorbant en forme de culotte est dans l'état déplié,
la région de faible masse surfacique (40) étant une région dans laquelle la masse surfacique de la partie centrale absorbante est inférieure à celle dans une région environnante et qui s'étend le long d'au moins une direction parmi la direction verticale et la direction latérale.

11. Article absorbant en forme de culotte selon la revendication 10, dans lequel
quand l'article absorbant en forme de culotte est dans l'état déplié et dans un état étiré,
une zone totale des régions de faible masse surfacique (40) se trouvant dans la partie de la partie centrale absorbante qui chevauche la partie d'extension (30E)
est supérieure à
une zone totale des régions de faible masse surfacique (40) se trouvant dans la partie de la partie centrale absorbante qui chevauche la partie au niveau de la taille.

12. Article absorbant en forme de culotte selon la revendication 10 ou la revendication 11, dans lequel
la région de faible masse surfacique (40) a une région allongée latéralement (42) qui est plus longue dans la direction latérale que dans la direction verticale, et
la région allongée latéralement (42) se trouve dans une partie de la partie centrale absorbante qui chevauche une partie arrière au niveau de la taille quand l'article absorbant en forme de culotte est dans l'état déplié.

13. Article absorbant en forme de culotte selon l'une quelconque des revendications 10 à 12, dans lequel
la région de faible masse surfacique (40) a une région allongée latéralement (42) qui est plus longue dans la direction latérale que dans la direction verticale, et
les régions d'une pluralité de régions allongées latéralement (42) se trouvent côte à côte dans la direction verticale dans une partie de la partie centrale absorbante qui chevauche une partie avant au niveau de la taille quand l'article absorbant en forme de culotte est dans l'état déplié.

14. Article absorbant en forme de culotte selon l'une quelconque des revendications 1 à 13, dans lequel
le corps principal absorbant (10) a une partie exposée sous une partie avant de l'élément extérieur (30) et sous une partie arrière de l'élément extérieur (30),
la partie exposée (10E) ayant une surface côté non orienté vers la peau qui est exposée vers l'extérieur, et
la région de faible masse surfacique (40) est mise en œuvre à la verticale et enfourche une extrémité inférieure d'au moins l'une parmi la partie avant de l'élément extérieur (30) et la partie arrière de l'élément extérieur.
